# EUROPEAN PATENT APPLICATION

(11) **EP 2 693 353 A2**
(43) Date of publication of application: **05.02.2014**
(21) Application number: 13159860.9
(22) Date of filing: 19.03.2013
(51) Int. Cl.: G06F 19/00

(54) **Method of generating user interface and apparatus for generating user interface by using the same**

(30) Priority: 01.08.2012 KR 20120084587
(71) Applicant: Samsung Electronics Co., Ltd, Gyeonggi-do 443-742 (KR)
(72) Inventor: Kim, Dong-woo, Seoul (KR); Kim, Ji-eun, Gyeonggi-do (KR); Park, Jeong-je, Gyeonggi-do (KR); Lee, Kwang-hyeon, Seoul (KR)
(74) Representative: D'Halleweyn, Nele Veerle Trees Gertrudis

(57) **Abstract**

An apparatus for generating a user interface image for indicating a patient's health-related information includes an information receiver for receiving the patient's health-related information, an information arranger and classifier for arranging at least one piece of information included in the health-related information in a time sequence, and a user interface (UI) generator for generating the user interface image for displaying the at least one piece of information that is arranged in a time sequence, along a first axis, whereby a patient and a doctor may easily recognize the health-related information.

## Description

### CROSS-REFERENCE TO RELATED APPLICATIONS

This application claims the benefit of Korean Patent Application No. 10-2012-0084587, filed on August 1, 2012, in the Korean Intellectual Property Office, the disclosure of which is incorporated herein in its entirety by reference.

### BACKGROUND

### 1. Field

One or more embodiments relate to a method of generating a user interface and an apparatus for generating a user interface by using the method, and more particularly, to a method of generating a user interface for recognizing a patient's personal health-related record and a health-related history and an apparatus for generating a user interface by using the method.

### 2. Description of the Related Art

Methods and apparatus for writing and using a personal health record (PHR) have been continually developed. The methods and apparatus for writing and using a PHR have focused on methods of writing, collecting, and reading a PHR.

For example, methods of establishing and reading a PHR have been suggested in connection with a pharmacy prescription management system or a patient's medical record system, such as an electronic medical record (EMR) or an electronic health record (EHR) system in hospitals or clinics. These conventional methods are provided to establish a PHR and to simply read predetermined information. Thus, even if the established PHR is read, it is difficult to easily recognize the history and characteristics of the PHR.

Accordingly, there is a need for patients or doctors to easily recognize a patient's health history, management history, or prescription history, based on a PHR.

### SUMMARY

Additional aspects and/or advantages of one or more embodiments will be set forth in part in the description which follows and, in part, will be apparent from the description, or may be learned by practice of one or more embodiments of disclosure. One or more embodiments are inclusive of such additional aspects.

One or more embodiments relate to a method of generating a user interface whereby a patient and a doctor may easily recognize a patient's health-related history, such as a health history, a management history, or a prescription history, and an apparatus for generating a user interface by using the method.

In detail, one or more embodiments relate to a method of generating a user interface whereby a patient and a doctor may easily recognize a patient's prescription history corresponding to a time sequence by using a patient's health-related information, and an apparatus for generating a user interface by using the method.

According to an aspect of one or more embodiments, there is provided an apparatus for generating a user interface image for indicating a patient's health-related information, the apparatus possibly including, for example: an information receiver for receiving the patient's health-related information; an information arranger and classifier for arranging at least one piece of information included in the health-related information in a time sequence; and a user interface (UI) generator for generating the user interface image for displaying the at least one piece of information that is arranged in a time sequence, along a first axis.

The health-related information may include a prescription of the patient.

The information arranger and classifier may classify a plurality of pieces of information included in the prescription into higher-order information and lower-order information that is included in the higher-order information.

The UI generator may generate the user interface image indicating a plurality of pieces of the lower-order information that are classified and arranged for respective pieces of the higher-order information along a second axis.

The higher-order information may include, for example, at least one of a disease management code, a disease, a hospital that issues the prescription, a type of hospital that issues the prescription, a patient's family, and a residential area of the patient when the health-related information is generated.

The lower-order information may include at least one prescribed medicine included in the prescription.

The information arranger and classifier may classify the at least one prescribed medicine included in the prescription according to disease, and the UI generator may generate the user interface image in which the disease and the prescribed medicine corresponding thereto are arranged and displayed along a second axis.

The information arranger and classifier may classify at least one prescribed medicine included in the prescription according to disease and a medicine type corresponding thereto, and the UI generator may generate the user interface image in which a disease, a medicine type corresponding to the disease, and prescribed medicine corresponding to the medicine type are arranged and displayed along the second axis.

The UI generator may generate the user interface image for displaying information indicating at least one of a patient's evaluation and a doctor's evaluation on the prescribed medicine.

The UI generator may generate the user interface image including a change menu key for changing the higher-order information that is arranged along the second axis.

The apparatus may further include an input/output unit for displaying the user interface image.

According to another aspect of one or more embodiments, there is provided a method of generating a user interface image for indicating a patient's health-related history, the method possibly including, for example: receiving the patient's health-related information; arranging at least one piece of information included in the health-related information in a time sequence; and generating the user interface image for displaying the at least one piece of information that is arranged in a time sequence, along a first axis.

### BRIEF DESCRIPTION OF THE DRAWINGS

These and/or other aspects will become apparent and more readily appreciated from the following description of embodiments, taken in conjunction with the accompanying drawings of which:
FIG. 1 is a block diagram of a user interface generating apparatus according to one or more embodiments;
FIG. 2 is a block diagram of a user interface generating apparatus according to one or more embodiments;
FIG. 3 is a diagram of a user interface image generated by a user interface (UI) generating device, according to one or more embodiments
FIG. 4 is a diagram of a user interface image generated by a UI generating device, according to one or more embodiments;
FIG. 5 is a diagram of a user interface image generated by a UI generating device, according to one or more embodiments;
FIG. 6 is a diagram of a user interface image generated by a UI generating device, according one or more embodiments;
FIG. 7 is a diagram of a user interface image generated by a UI generating device, according to one or more embodiments;
FIG. 8 is a diagram of a user interface image generated by a UI generating device, according to another one or more embodiments n; and
FIG. 9 is a flowchart of a user interface generating method according to one or more embodiments.

### DETAILED DESCRIPTION

Reference will now be made in detail to one or more embodiments, illustrated in the accompanying drawings, wherein like reference numerals refer to like elements throughout. In this regard, embodiments of the present invention may be embodied in many different forms and should not be construed as being limited to embodiments set forth herein, as various changes, modifications, and equivalents of the systems, apparatuses and/or methods described herein will be understood to be included in the invention by those of ordinary skill in the art after embodiments discussed herein are understood. Accordingly, embodiments are merely described below, by referring to the figures, to explain aspects of the present invention.

Hereinafter, a method of generating a user interface and an apparatus of generating a user interface by using the same will be described with regard to one or more exemplary embodiments with reference to the attached drawings. Expressions such as "at least one of," when preceding a list of elements, modify the entire list of elements and do not modify the individual elements of the list.

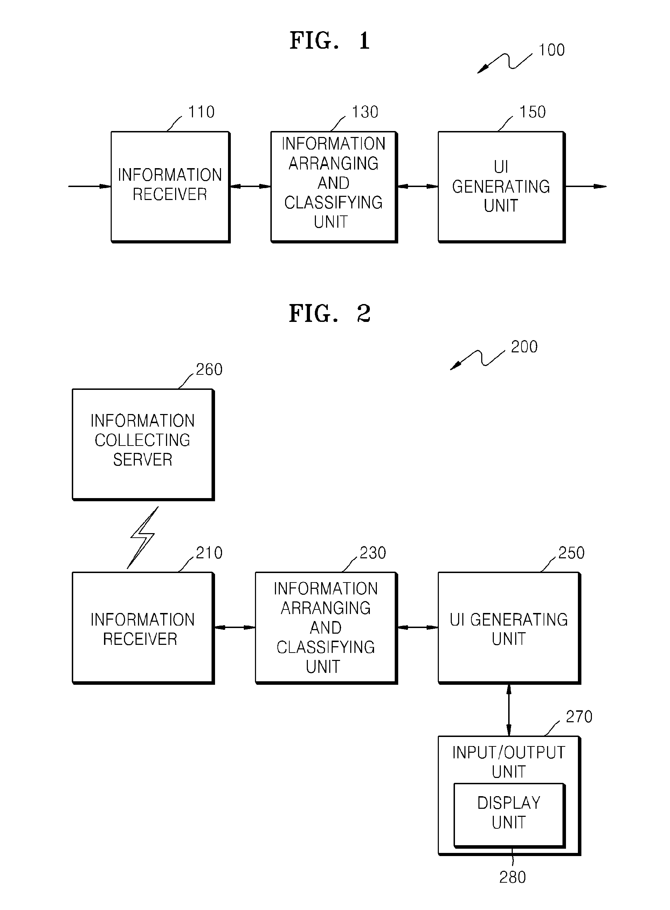

FIG. 1 is a block diagram of a user interface generating apparatus 100 according to one or more embodiments.

Referring to FIG. 1, the user interface generating apparatus 100 is an apparatus for generating a user interface image for indicating a patient's health-related history. The user interface generating apparatus 100 may include an information receiver 110, an information arranger and classifier 130, and a user interface (UI) generator 150.

The information receiver 110 may receive the patient's health-related information. The information receiver 110 may receive health-related information of respective patients and may classify, receive, and store the health-related histories of the respective patients. Here, a health-related information may include, for example, information by which a patient or a doctor may recognizes the patient's treatment history or prescription history. In detail, the health-related information may include a patient's prescription.

A prescription is an administration instruction that is written by a doctor in order to administer predetermined medicine according to a patient's symptoms and disease after the doctor treats the patient in a hospital or a clinic. The prescription may include, for example, a disease classification code, information about prescribed medicine (e.g., a name of prescribed medicine), a name of a medical institution, information about a doctor who wrote the prescription, and so on.

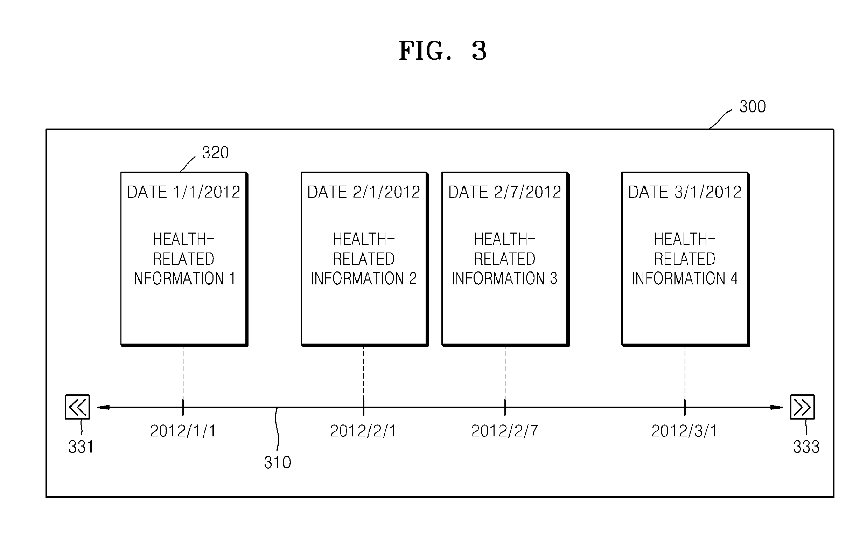

The information arranger and classifier 130 may arrange at least one piece of information included in the health-related information received by the information receiver 110 in a time sequence. An operation of classifying information in the information arranger and classifier 130 is described in detail with reference to FIGS. 3 to 8.

The UI generator 150 may generate a user interface image for displaying the at least one piece of information that is arranged in a time sequence, along a first axis. In detail, the UI generator 150 may generate the user interface image for arranging and displaying a plurality of pieces of information included in the health-related information, which are arranged in a time sequence by the information arranger and classifier 130, in a horizontal or vertical direction.

The information arranger and classifier 130 may classify a plurality of pieces of information included in the prescription into higher-order information and lower-order information that is included in the higher-order information. In detail, the higher-order information refers to the information which is comprehensive of other information from among the plurality of pieces of information included in the prescription. In detail, the higher-order information may include, for example, at least one of a disease management code, disease, a hospital that issues a prescription, a type of hospital that issues a prescription, a patient's family, and a residential area of the patient, when the health-related information is generated.

The type of hospital may be classified according to a medical department such as, for example, internal medicine, pediatrics, or dermatology. In addition, the type of hospital may be classified, for example, into a primary medical institution for treating a single field, a secondary medical institution that has specialists and four or more medical fields, and a tertiary medical institution such as a university hospital.

Disease management codes may be recorded in a prescription and may be classified to recognize a disease. In addition, because the prescription may include a name and address of a medical institution that issues the prescription, a residential area of a patient may be recognized, when health-related information, that is, the prescription is generated. When the plurality of pieces of information included in the prescription are classified by using a residential area of the patient as higher-order information and the user interface image including the classified information is generated, the generated user interface image may be used to determine whether a previous disease of the patient is based on an endemic disease that is frequently caused in a predetermined area.

In addition, when the plurality of pieces of information included in the prescription that correspond to each member of the patient's family are classified by using the patient's family as higher-order information and the user interface image including the classified information is generated, the generated user interface image may be used to determine whether the disease is due to a patient's family history or heredity.

Classified lower-order information included in the higher-order information may include at least one prescribed medicine included in the prescription. For example, if the higher-order information is a disease, the lower-order information of the disease may be the prescribed medicine corresponding to the disease.

The UI generator 150 may generate the user interface image indicating a plurality of pieces of lower-order information that are classified for respective pieces of higher-order information and arranged along a second axis. For example, the UI generator 150 may generate the user interface image in which a horizontal direction denotes a time sequence and a vertical direction denotes the lower-order information that is classified and arranged for respective pieces of higher-order information.

FIG. 2 is a block diagram of a user interface generating apparatus 200 according to one or more embodiments. With regard to the user interface generating apparatus 200 shown in FIG. 2, an information receiver 210, an information arranger and classifier 230, and a UI generator 250 are the same as the information receiver 110, the information arranger and classifier 130, and the UI generator 150 shown in FIG. 1, respectively. Thus, a description that is the same as the description of the user interface generating apparatus 100 in FIG. 1 is omitted.

The user interface generating apparatus 200 may further include at least one of an information collecting server 260 and an input/output unit 270, compared with the user interface generating apparatus 100 of FIG. 1.

The information collecting server 260 may collect a patient's health-related information. In detail, the information collecting server 260 may collect health-related information that is classified for each patient. The health-related information may correspond for example, to the above-described prescription as well as various medical records stored in a hospital, a personal health record (PHR), or an electronic health record (EHR). In addition, the information collecting server 260 may accumulate health-related information for a predetermined period of time from a current point in time, such as past years to collect the health-related information for each patient.

The information collecting server 260 may be connected to the information receiver 210 through a predetermined wired or wireless network. The information receiver 210 may periodically update and receive the health-related information for each patient from the information collecting server 260. In addition, whenever the information collecting server 260 collects the health-related information for each patient, the information collecting server 260 may transmit the collected information to the information receiver 210.

The input/output unit 270 may display the user interface image generated by the UI generator 250. In detail, the input/output unit 270 may include a display unit 280. The display unit 280 may display the user interface image generated by the UI generator 250. In addition, the input/output unit 270 may be configured in a combination form with a user input means, e.g. a touch screen. Thus, a user instruction or request may be received through the touch screen and may be transmitted to the UI generator 250 or the information arranger and classifier 230.

Hereinafter, exemplary embodiments of a user interface image generated by the UI generators 150 and 250 in FIGS. 1 and 2 will be described with reference to FIGS. 3 to 8. In addition, a case where the user interface image shown in FIGS. 3 to 8 is generated by the user interface generating apparatus 100 of FIG. 1 will be described.

FIG. 3 is a diagram of a user interface image 300 generated by a UI generating device, according to one or more embodiments.

The user interface image 300 may display at least one piece of information included in health-related information and aligned in a time sequence at the information arranger and classifier 130 along a first axis 310. FIG. 3 shows a case where the first axis 310 denotes a horizontal axis and has a time unit of 'year/month/day'.

In addition, the user interface image 300 may include horizontal-axis movement menu keys 331 and 333 for moving a time sequence indicated along the first axis 310. A user such as a patient or a doctor may easily recognize a patient's health-related information in a time sequence by using the horizontal-axis movement menu keys 331 and 333.

In FIG. 3, a health-related information 1 320 collected at a predetermined time '2012/1/1 (January 1, 2012)' may be a prescription itself. In addition, the UI generator 150 may include only information about a name of a predetermined disease included in the prescription and prescribed medicine corresponding to the predetermined disease in health-related information 1 320.

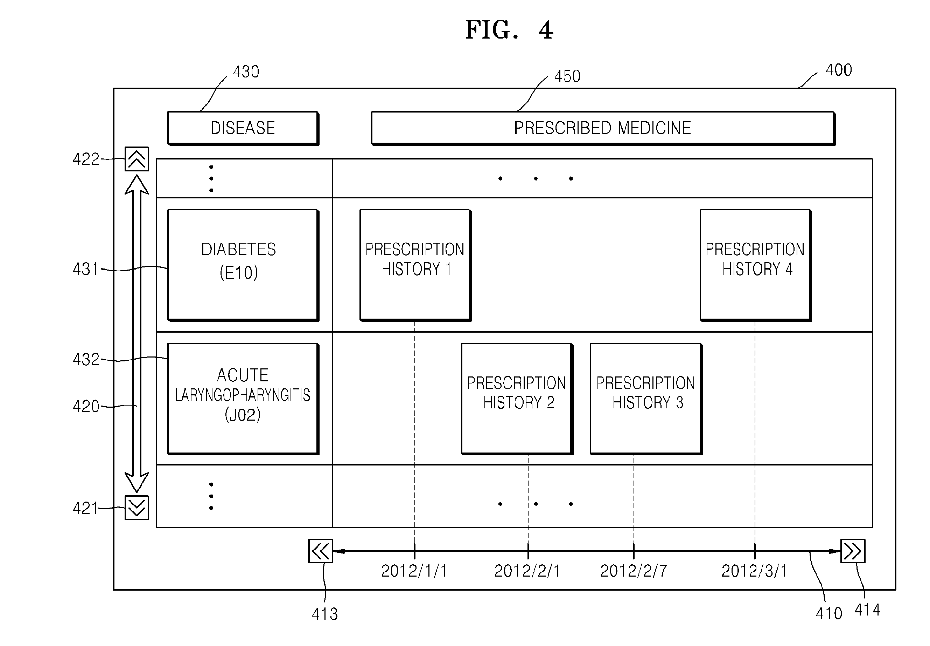

FIG. 4 is a diagram of a user interface image 400 generated by a UI generating device, according to one or more embodiments.

Referring to FIG. 4, the user interface image 400 may indicate a time along a horizontal axis that is a first axis 410. The user interface image 400 has a time unit of 'year/month/day' along the horizontal axis that is the first axis 410, like the user interface image 300 shown in FIG. 3.

Hereinafter, a case where health-related information received by the information receiver 110 includes a prescription in order to generate user interface images shown in FIGS. 4 to 8 will be described.

The information arranger and classifier 130 may classify at least one prescribed medicine included in the prescription by each disease. The disease may be classified by using a disease classification code included in the prescription. That is, a plurality of pieces of information included in the prescription may be classified according to disease as the above-described higher-order information and prescribed medicines as the lower-order information. The UI generator 150 may generate the user interface image 400 in which diseases and prescribed medicines corresponding thereto are arranged and displayed along a vertical axis that is a second axis 420. In detail, a disease 430 and a prescribed medicine 450 corresponding to the disease 430 may be sequentially displayed, as shown in FIG. 4.

Referring to FIG. 4, the user interface image 400 may display a prescription history 1 on January 1, 2012, and a prescription history 4 on March 1, 2012, which correspond to diabetes 431, and a prescription history 2 on February 1, 2012, and a prescription history 3 on February 7, 2012, which correspond to acute laryngopharyngitis 432.

In addition, a disease classification code, for example, 'E10' that is written in brackets may be added to a disease name, for example, 'diabetes', and may be displayed.

In addition, vertical-axis movement menu keys 421 and 422 for moving diseases arranged along a vertical axis that is the second axis 420 in up and down directions may be added to constitute the user interface image 400. A user such as a patient or a doctor may easily recognize a prescription history of a target disease in a time sequence by using the horizontal-axis movement menu keys 413 and 414 and the vertical-axis movement menu keys 421 and 422.

For example, when a patient goes to the doctor because of acute laryngopharyngitis, the doctor may easily recognize a previous prescription history related to the acute laryngopharyngitis 432 in a time sequence and may check the previous prescription history so as to write a current prescription, with reference to the user interface image 400.

In addition, a prescription history for each disease, for example, the prescription history 1 may include, for example, information about medicine prescribed by a doctor according to disease, information about a side effect of the medicine, or information about stability of the medicine.

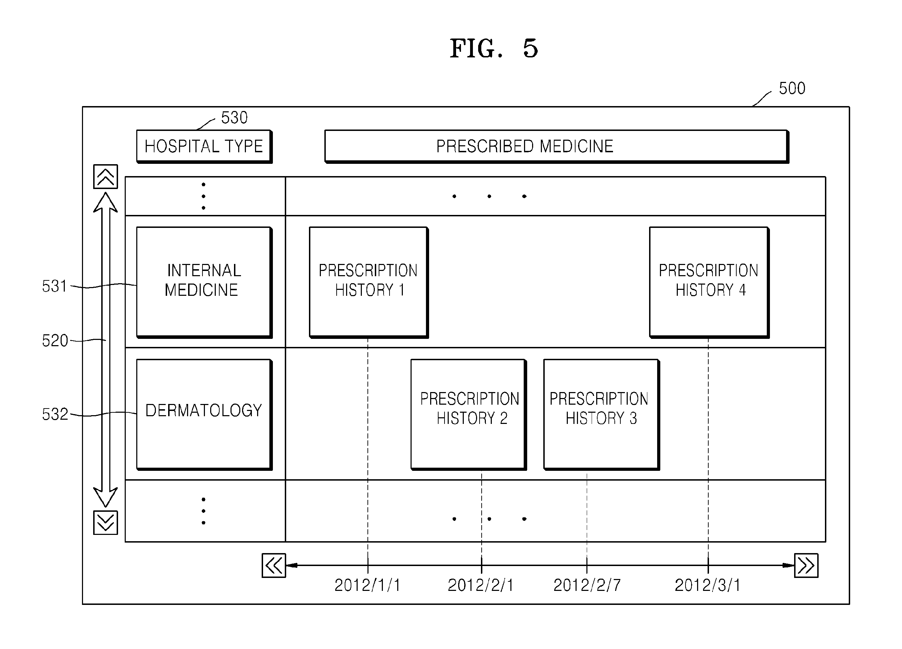

FIG. 5 is a diagram of a user interface image 500 generated by a UI generating device, according to one or more embodiments. The user interface image 500 of FIG. 5 may be the same as the user interface image 400 shown in FIG. 4, except that higher-order classification arranged along a second axis 520 is a 'hospital type', and thus, a repeated description of elements previously described with respect to FIG. 4 is omitted herein.

The information arranger and classifier 130 may obtain information about a hospital type from the received health-related information, for example, a prescription and may classify a prescription history according to the hospital type.

The UI generator 150 may generate the user interface image 500 that arranges and displays a hospital type and a prescription history corresponding thereto along the second axis 520.

In this regard, the hospital type may be classified according to a medical field such as internal medicine 531 or dermatology 532, for example. In addition, the hospital type may be classified, for example, into a primary medical institution, a secondary medical institution, and a tertiary medical institution, as described above.

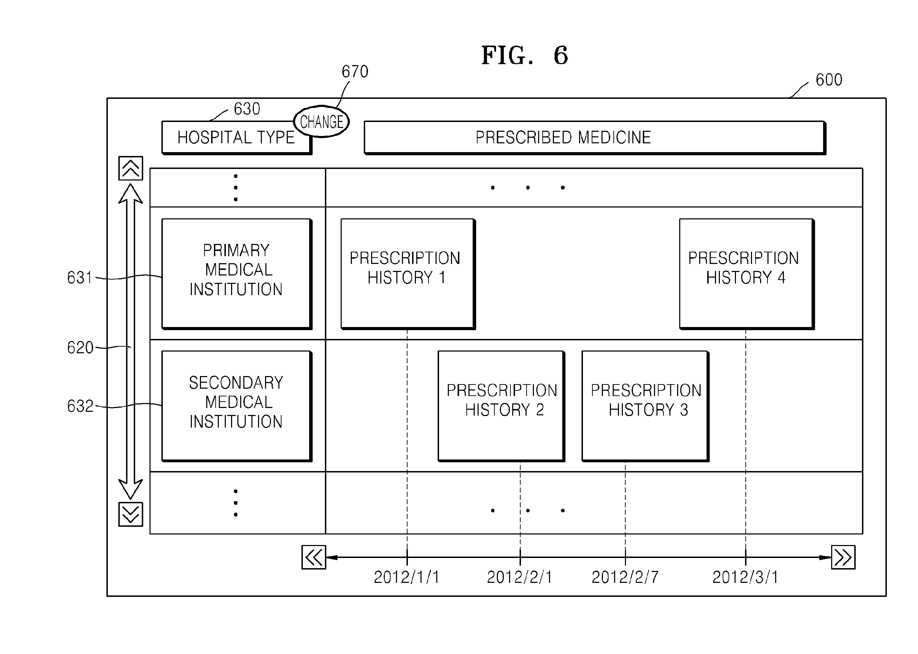

FIG. 6 is a diagram of a user interface image 600 generated by a UI generating device, according to one or more embodiments. The user interface image 600 may be the same as the user interface image 500 shown in FIG. 5, except that higher-order classification arranged along a second axis 620 is a hospital level among 'hospital types' for example, a primary medical institution 631, a secondary medical institution 632, or a tertiary medical institution (not shown), and a repeated description of elements previously described with respect to FIG. 5 is omitted herein.

In addition, the UI generator 150 may generate a user interface image including a change menu key 670 for changing higher-order information that is arranged along the second axis 620. In detail, as shown in FIG. 6, the UI generator 150 may generate the user interface image 600 including the change menu key 670 for changing a 'hospital type' 630 that is higher-order information to other higher-order information, for example, a disease management code, a disease name, a hospital name that issues the prescription, a patient's family, or a hospital type according to a medical field. For example, when a user presses the change menu key 670 with a remote controller or a touch screen, a menu list (not shown) for selecting any one of a plurality of pieces of higher-order information other than the 'hospital type' 630 that is current higher-order information may be generated, and the user may change the higher-order information arranged along the second axis 620 by using the generated menu list (not shown).

Accordingly, the user may easily recognize a patient's health-related history by using the user interface image 600 in which a plurality of pieces of information are arranged according to desired higher-order information and a desired sequence.

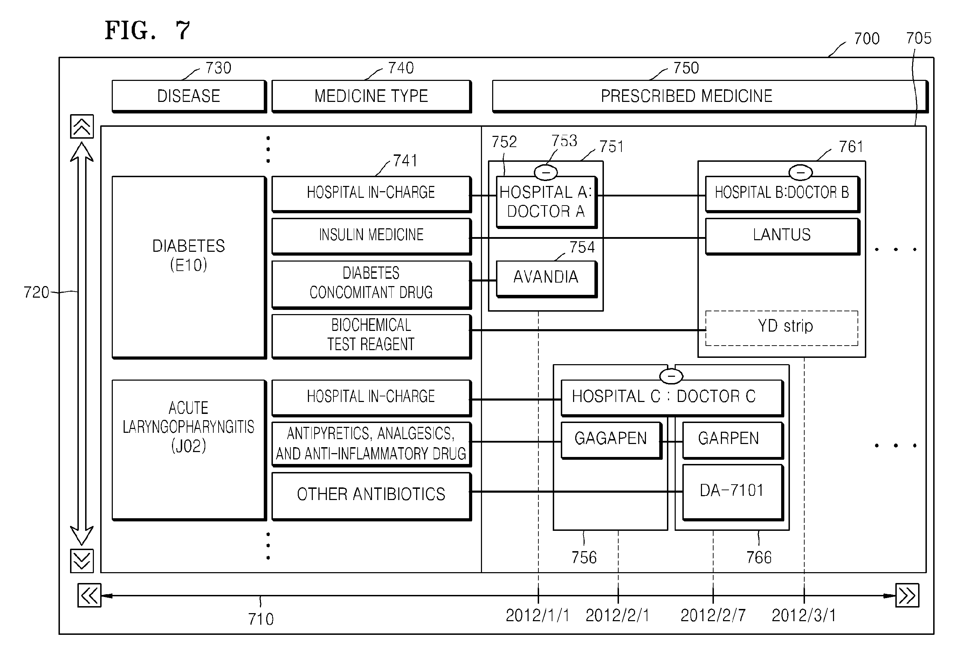

FIG. 7 is a diagram of a user interface image 700 generated by a UI generating device, according to one or more embodiments.

The information arranger and classifier 130 may classify a plurality of pieces of information included in a prescription in a sequence corresponding, for example, to a disease, a medicine type corresponding to the disease, and prescribed medicine corresponding to the medicine type.

In addition, the UI generator 150 may generate the user interface image 700 in which a predetermined disease, a medicine type corresponding to the predetermined disease, and prescribed medicine corresponding to the medicine type are arranged and displayed along a second axis 720.

Referring to FIG. 7, the user interface image 700 may arrange and display information about a disease 730, a medicine type 740 corresponding to the disease 730, and a prescribed medicine 750 corresponding to the medicine type 740 along a second axis 720. A first axis 710 is the same as the first axis 410 described with reference to FIG. 4, and thus, a detailed description thereof is omitted herein.

Referring to a region 705 of FIG. 7, the plurality of pieces of information included in the prescription may be classified into diabetes or acute laryngopharyngitis in a sequence of diseases. The plurality of pieces of information may be classified into a insulin medicine that is an injection, a diabetes concomitant drug taken by a patient, and a biochemical test reagent as medicine types corresponding to a predetermined disease, for example, diabetes. In addition, the user interface image 700 may also display information about a hospital 741 in-charge, which treats a predetermined disease, for example, diabetes.

In addition, the medicine type corresponding to acute laryngopharyngitis may be classified into 'antipyretics, analgesics, and anti-inflammatory drugs' and other antibiotics.

Avandia 754, a diabetes concomitant drug, and information 752 about a doctor A of a hospital A that is the hospital 741 in-charge may be displayed as a prescription history 1 751 generated on January 1, 2012. When a hospital in-charge is not required to be referred to, a user may remove an image of the hospital in-charge by using a display removal key 753 included in the user interface image 700.

The user interface image 700 may display a prescription history 2 756 prescribed on February 1, 2012, a prescription history 3 766 prescribed on February 7, 2012, and a prescription history 4 761 prescribed on March 1, 2012, in a time sequence.

The UI generator 150 may generate the user interface image 700 shown in FIG. 7 such that a user such as a doctor or a patient may easily recognize prescribed medicines in a time sequence according to disease and a medicine type corresponding thereto to rapidly and accurately recognize a patient's disease treatment history.

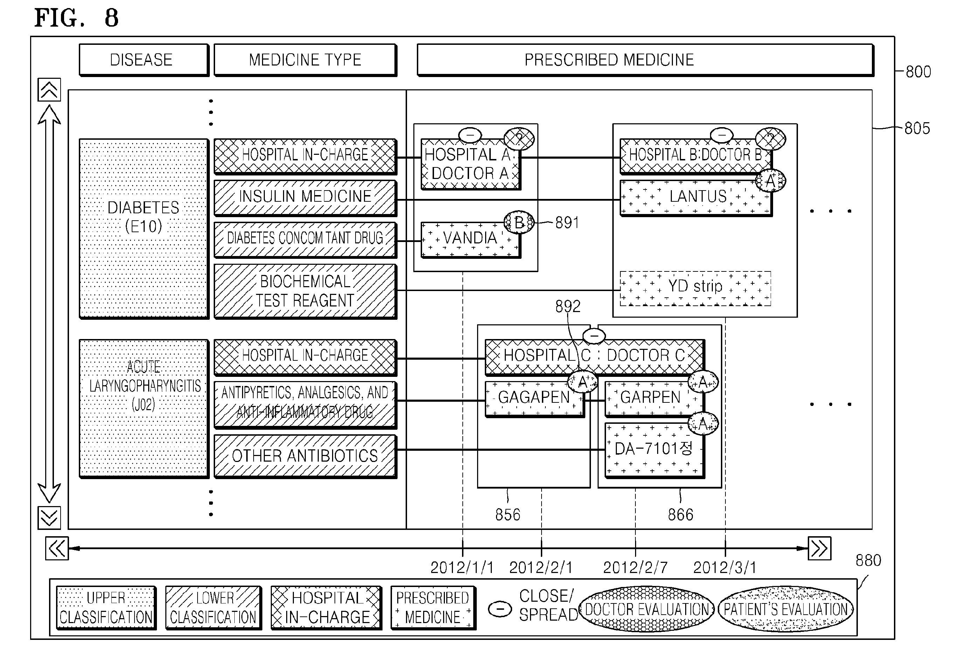

FIG. 8 is a diagram of a user interface image 800 generated by a UI generating device, according to one or more embodiments.

The user interface image 800 shown in FIG. 8 may be the same as the user interface image 700 show in FIG. 7, except for a region 880 and evaluation display keys 891 and 892 for respective prescribed medicines. Thus, a repeated description of elements previously described with respect to FIG. 7 is omitted herein.

In detail, the UI generator 150 may generate the user interface image 800 in which information indicating at least one of a patient's evaluation and a doctor evaluation's on a prescribed medicine is connected to the prescribed medicine and is displayed.

Referring to FIG. 8, the evaluation display key 891 of doctors may be displayed together with a prescribed medicine, for example, 'Avandia' that is classified as a diabetes concomitant drug. Doctors may determine whether corresponding medicine is appropriate to treat a patient's disease in consideration of whether there is a side effect or a degree of improving a patient's symptom and may indicate the determination result as a grade or a score. For example, the doctors may determine medicines as respective grades, and for example, may determine medicine having no side effect and an excellent degree of improving a patient's symptom as a grade A, may determine medicine having a side effect and an average degree of improving a patient's symptom as a grade B, and may determine medicine having a side effect as a grade F.

In this regard, the evaluation display key 891 of doctors may include information indicating the above-described determination result.

FIG. 8 shows a case where the evaluation display key 891 of doctors includes grade information such as A, B or F.

Referring to FIG. 8, the evaluation display key 892 of a patient may also be displayed with regard to a prescribed medicine, for example, 'gagapen' that is classified as an antipyretic, analgesic, and anti-inflammatory drug. The patient may determine a degree of being suitable for corresponding medicine in consideration of whether there is a side effect and a degree of improving a symptom as a grade or a score.

The evaluation display key 892 of the patient may include information indicating the above-described determination result of the patient. FIG. 8 shows a case where the evaluation display key 892 of the patient includes grade information such as A or B.

Referring to FIG. 8, information included in the user interface image 800, which is categorized in the same classification, may be displayed with the same color or shape. For example, diabetes and acute laryngopharyngitis, which are diseases, may be displayed by filling corresponding spaces with the same color. In addition, the user interface image 800 may include a menu 880 for explaining classification of information included in the user interface image 800.

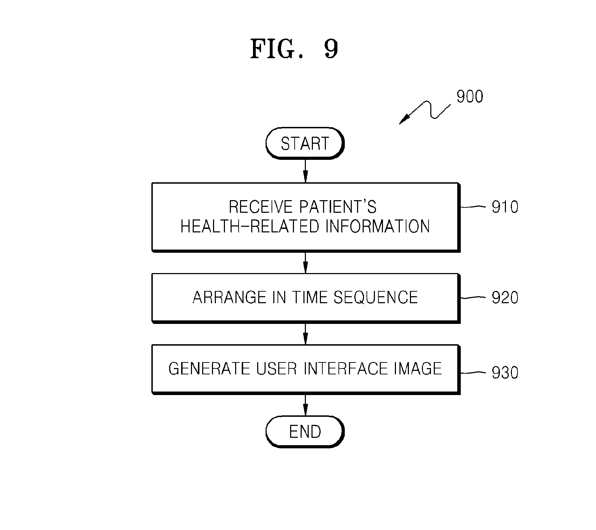

FIG. 9 is a flowchart of a user interface generating method 900 according to one or more embodiments.

The user interface generating method 900 according to the present embodiment is a method of generating a user interface image indicating a patient's health-related history. The user interface generating method 900 according to the present embodiment may be performed by the user interface generating apparatuses 100 and 200 that have been described with reference to FIGS. 1 to 8. Hereinafter, the user interface generating method 900 will be described with reference to the user interface generating apparatus 100 that has been described with reference to FIG. 1.

Referring to FIG. 9, the user interface generating method 900 may include receiving a patient's health-related information (operation 910). Operation 910 may be performed by the information receiver 110.

The patient's health-related information received in operation 910 may include information indicating a previous patient treatment history or a prescription history. In detail, the patient's health-related information received in operation 910 may include, for example, a patient's prescription.

At least one piece of information included in the patient's health-related information received in operation 910 may be arranged in a time sequence (operation 920). Operation 920 may be performed by the information arranger and classifier 130. In detail, the user interface image for arranging and displaying a plurality of pieces of information included in the health-related information, which may be arranged in a time sequence by the information arranger and classifier 130, in a horizontal or vertical direction may be generated.

In addition, the user interface image displaying at least one piece of information that is arranged in a time sequence, along a first axis may be generated (operation 930). Operation 930 may be performed by the UI generator 150.

The user interface generating method 900 may further include classifying information included in a patient's prescription into higher-order information and lower-order information included in the higher-order information (not shown). The classifying operation of the higher-order information and the lower-order information may be performed by the information arranger and classifier 130.

The user interface generating method 900 may further include classifying at least one prescribed medicine included in a prescription according to disease (not shown). The classifying operation according to disease may be performed by the information arranger and classifier 130. In this case, the generating of the user interface image may further include generating the user interface image in which a predetermined disease and prescribed medicine corresponding thereto are arranged and displayed along a second axis.

In addition, the classifying operation according to disease may include classifying at least one prescribed medicine included in the prescription according to disease and a medicine type corresponding thereto (not shown). The classifying operation according to disease and a medicine type corresponding thereto may be performed by the information arranger and classifier 130. In this case, the generating (operation 930) of the user interface image may further include generating the user interface image in which a disease, a medicine type corresponding to the disease, and prescribed medicine corresponding to the medicine type are aligned and displayed along the second axis.

The user interface generating method 900 may further include displaying the user interface image generated in operation 930 such that a user may visibly recognize the user interface image (not shown). The displaying operation may be performed by the input/output unit 270.

The user interface generating method 900 described with reference to FIG. 9 may include the same operations of the user interface generating apparatuses 100 and 200 that have been described with reference to FIGS. 1 to 8. Accordingly, a repeated description of elements previously described with respect to FIGS. 1 to 8 is omitted herein.

In one or more embodiments, any apparatus, system, element, or interpretable unit descriptions herein include one or more hardware devices or hardware processing elements. For example, in one or more embodiments, any described apparatus, system, element, retriever, pre or post-processing elements, tracker, detector, encoder, decoder, etc., may further include one or more memories and/or processing elements, and any hardware input/output transmission devices, or represent operating portions/aspects of one or more respective processing elements or devices. Further, the term apparatus should be considered synonymous with elements of a physical system, not limited to a single device or enclosure or all described elements embodied in single respective enclosures in all embodiments, but rather, depending on embodiment, is open to being embodied together or separately in differing enclosures and/or locations through differing hardware elements.

In addition to the above described embodiments, embodiments can also be implemented through computer readable code/instructions in/on a non-transitory medium, e.g., a computer readable medium, to control at least one processing device, such as a processor or computer, to implement any above described embodiment. The medium can correspond to any defined, measurable, and tangible structure permitting the storing and/or transmission of the computer readable code.

The media may also include, e.g., in combination with the computer readable code, data files, data structures, and the like. One or more embodiments of computer-readable media include: magnetic media such as hard disks, floppy disks, and magnetic tape; optical media such as CD ROM disks and DVDs; magneto-optical media such as optical disks; and hardware devices that are specially configured to store and perform program instructions, such as read-only memory (ROM), random access memory (RAM), flash memory, and the like. Computer readable code may include both machine code, such as produced by a compiler, and files containing higher level code that may be executed by the computer using an interpreter, for example. The media may also be any defined, measurable, and tangible distributed network, so that the computer readable code is stored and executed in a distributed fashion. Still further, as only an example, the processing element could include a processor or a computer processor, and processing elements may be distributed and/or included in a single device.

The computer-readable media may also be embodied in at least one application specific integrated circuit (ASIC) or Field Programmable Gate Array (FPGA), as only examples, which execute (e.g., processes like a processor) program instructions.

While aspects of the present invention has been particularly shown and described with reference to differing embodiments thereof, it should be understood that these embodiments should be considered in a descriptive sense only and not for purposes of limitation. Descriptions of features or aspects within each embodiment should typically be considered as available for other similar features or aspects in the remaining embodiments. Suitable results may equally be achieved if the described techniques are performed in a different order and/or if components in a described system, architecture, device, or circuit are combined in a different manner and/or replaced or supplemented by other components or their equivalents.

Thus, although a few embodiments have been shown and described, with additional embodiments being equally available, it would be appreciated by those skilled in the art that changes may be made in these embodiments without departing from the principles and spirit of the invention, the scope of which is defined in the claims and their equivalents.

## Claims

1. An apparatus generating a user interface image indicating a patient's health-related information, the apparatus comprising:
an information receiver receiving the patient's health-related information;
an information arranger and classifier arranging in a time sequence, using at least one processor, at least one piece of information included in the health-related information; and
a user interface (UI) generator generating the user interface image displaying the at least one piece of information that is arranged in a time sequence, along a first axis.

2. The apparatus of claim 1, wherein the health-related information comprises a prescription of the patient.

3. The apparatus of claim 2, wherein the information arranger and classifier classifies a plurality of pieces of information included in the prescription into higher-order information and lower-order information included in the higher-order information.

4. The apparatus of claim 3, wherein the UI generator generates the user interface image indicating a plurality of pieces of the lower-order information that are classified and arranged according to respective pieces of the higher-order information along a second axis.

5. The apparatus of claim 3, wherein the higher-order information comprises at least one of a disease management code, a disease, a hospital that issues the prescription, a type of hospital that issues the prescription, a patient's family, and a residential area of the patient when the health-related information is generated.

6. The apparatus of claim 3, wherein the lower-order information comprises at least one prescribed medicine included in the prescription.

7. The apparatus of claim 2, wherein the information arranger and classifier classifies the at least one prescribed medicine included in the prescription according to disease, and
wherein the UI generator generates the user interface image in which the disease and the prescribed medicine corresponding thereto are arranged and displayed along a second axis.

8. The apparatus of claim 7, wherein the information arranger and classifier classifies at least one prescribed medicine included in the prescription according to a disease and a medicine type corresponding to the disease, and
wherein the UI generator generates the user interface image in which the disease, the medicine type corresponding to the disease, and the prescribed medicine corresponding to the medicine type are arranged and displayed along the second axis.

9. The apparatus of claim 7, wherein the UI generator generates the user interface image displaying information indicating at least one of a patient's evaluation and a doctor's evaluation on the prescribed medicine.

10. The apparatus of claim 1, further comprising an input/output unit displaying the user interface image.

11. A method of generating a user interface image indicating a patient's health-related history, the method comprising:
receiving the patient's health-related information;
arranging in a time sequence, using at least one processor, at least one piece of information included in the health-related information; and
generating the user interface image displaying the at least one piece of information that is arranged in a time sequence, along a first axis.

12. The method of claim 11, wherein the health-related information comprises a prescription of the patient.

13. The method of claim 12, further comprising classifying a plurality of pieces of information included in the prescription into higher-order information and lower-order information included in the higher-order information.

14. The method of claim 13, wherein the generating of the user interface image comprises generating the user interface image indicating a plurality of pieces of the lower-order information that are classified and arranged according to respective pieces of the higher-order information along a second axis.

15. The method of claim 12, further comprising classifying the at least one prescribed medicine included in the prescription according to a disease,
wherein the generating of the user interface image further comprises generating the user interface image in which the disease and the prescribed medicine corresponding to the disease are arranged and displayed along a second axis.
